# EUROPEAN PATENT APPLICATION

(11) **EP 4 495 257 A1**
(43) Date of publication of application: **22.01.2025**
(21) Application number: 23792240.6
(22) Date of filing: 21.04.2023
(51) Int. Cl.: C12Q 1/6876, C12Q 1/6825, C12Q 1/70, C12N 15/113, C12N 9/22

(54) **MICROPARTICLE PROBE FOR SINGLE NUCLEOTIDE POLYMORPHISM ANALYSIS**

(30) Priority: 21.04.2022 KR 20220049310
(71) Applicant: Ezdiatech Inc., Chungcheongnam-do 31116 (KR)
(72) Inventor: JUNG, Yong-Gyun, Seoul 05792 (KR)
(74) Representative: Prüfer & Partner mbB Patentanwälte · Rechtsanwälte
(86) International application number: PCT/KR2023/005460
(87) International publication number: WO 2023/204667

(57) **Abstract**

The present invention relates to a Microparticle probe for single nucleotide polymorphisms. The present invention provides a Microparticle probe for single nucleotide polymorphisms comprising a microparticle; capture probes that are introduced to the surface of the microparticle and contain a complementary sequence to a target nucleic acid; and reporter nucleic acids that are introduced to the surface of the microparticle and generate a signal by an external stimulus.

## Description

### [Technical Field]

The present invention relates to a Microparticle probe for single nucleotide polymorphisms.

### [Background Art]

The genetic codes contained in the genomes of biological organisms belonging to the same species are not identical to each other, and differences in the nucleotide sequences, called polymorphisms, are known. Deletion or insertion of 1 to 10 nucleotide(s), duplication of a specific nucleotide sequence, etc. are known as polymorphisms. If a single nucleotide is replaced by another nucleotide, it is called single nucleotide (base) polymorphisms (SNP).

The single nucleotide polymorphisms (SNPs) refer to changes in one DNA base sequence and are involved in numerous genetic traits. SNPs are distributed with a probability of 1 per about 1,000 base sequences and are the main cause of differences in genetic disease incidence and drug resistance between individuals. SNPs are divided into a total of four types (C:G->T:A, C:G->G:C, C:A->G:T, T:A->A:T) based on changes in the four bases (A, G, C, T). Among these, C/T type SNPs, which are polymorphisms between cytosine (C) and thymine (T), are found in approximately 70% of the total.

As the importance of SNP analysis has increased for reasons such as individual disease prediction and personalized medicine, efficient detection methods for SNPs have been continuously studied, and already, numerous methods for identifying SNPs have been developed. Representative examples comprise the single-strand conformation polymorphism (SSCP) analysis and mini-sequencing methods as an allele-nonspecific method, and the RT-PCR method using molecular beacons and ligase analysis as an allele-specific method.

Over the past 20 years, many SNP screening methods have been developed, but a ligase-based SNP analysis method has been suggested as a promising method in terms of the efficiency of analysis. This method has several advantages. First, it enables multiplex analysis, and can be combined with trace analysis methods such as flow cytometry, micro array, micro fluidics, capillary, etc. to simultaneously perform SNP analysis on various samples. In addition, it can be easily applied in combination with methods such as using nanoparticles such as gold nanoparticles to increase signal amplification and specificity, or with optical imaging methods using FRET. In particular, if ligation analysis is linked to PCR, it is possible to obtain a high signal even with a small concentration of sample, and it is possible to integrate with existing DNA-based automated systems. However, T4 DNA ligase and Thermal DNA ligase have a fundamental problem in securing the ligation result that distinguishes G:C match and G:T mismatch at the terminal part of the ligation junction. That is, the method of distinguishing SNPs through the success or failure of ligation is fast and simple, and can be applied in conjunction with various methods, but there is a problem that false positive results exist, which cause a reaction especially in G:T mismatch during ligase reaction. That is, DNA ligase inevitably shows positive results even in G:T mismatch, which is a non-complementary bond, and ultimately, these inaccurate results are the biggest factor in lowering the reliability of the system that uses ligase to determine C/T type SNPs. Deng's research team published the results of ligation-based SNP analysis based on a DNA chip using T4 DNA ligase, but their results also showed false positive errors in which G:T mismatches were ligated.

Therefore, there is a need for a new method of SNP analysis that can confirm ligation reactions for G:C matches and G:T mismatches without error.

### [Disclosure]

### [Technical Problem]

In order to solve the above-mentioned problems, the present invention aims to provide a Microparticle probe for single nucleotide polymorphisms.

### [Technical Solution]

In order to solve the above-mentioned problems, the present invention provides a Microparticle probe for single nucleotide polymorphisms comprising a microparticle; capture probes that are introduced to the surface of the microparticle and contain complementary sequences to a target nucleic acid; and reporter nucleic acids that are introduced to the surface of the microparticle and generate a signal by an external stimulus.

In one embodiment, the capture probes may comprise a guide RNA.

In one embodiment, the guide RNA may comprise CRISPR RNA (crRNA) having a nucleotide sequence capable of hybridizing with a target site of a gene and trans-activating crRNA (tracrRNA).

In one embodiment, the guide RNA binds to the target nucleic acid and when it meets a lyase, may cleave the target nucleic acid and the reporter nucleic acids.

In one embodiment, the reporter nucleic acids may be a nucleic acid labeled with a luminous substance.

In one embodiment, the reporter nucleic acids may be a base sequence to be cut when used in a gene scissors system.

In one embodiment, the microparticle may contain magnetic particles inside.

In one embodiment, the microparticle may have a core-shell structure having a core containing a magnetic substance and a shell layer having a uniform thickness surrounding the core.

In one embodiment, the microparticle may have a size and specific gravity that does not float in water.

The present invention also provides a kit for analyzing single base polymorphisms comprising a Microparticle probe for single nucleotide polymorphisms, a lyase, and a reagent for nucleic acid amplification.

The present invention also provides a method for analyzing single base polymorphisms comprising the steps of: (a) extracting a target nucleic acid for analyzing single base polymorphisms from a specimen; (b) amplifying the target nucleic acid; (c) providing a Microparticle probe for single nucleotide polymorphisms comprising a microparticle, a guide RNA that is introduced to the surface of the microparticle and contain complementary sequences to the target nucleic acid, and reporter nucleic acids that are introduced to the surface of the microparticle and generate a signal by an external stimulus; (d) reacting the guide RNA of the microparticle for analyzing single base polymorphisms with the target nucleic acid to form a complex of the Microparticle probe for single nucleotide polymorphisms and the target nucleic acid; (e) binding lyase to the guide RNA of the microparticle for analyzing single base polymorphisms; (f) cleaving each base sequence of the target nucleic acid and the reporter nucleic acids by an activation reaction of the lyase; and (g) detecting the target nucleic acid by measuring the on-off of the signal emitted from the complex by the external stimulus.

In one embodiment, the detection of the target nucleic acid can be done by using two or more types of microparticle probes for analyzing single base polymorphisms and measuring the difference in luminescence of each probe to identify the presence or absence of the target nucleic acid.

In one embodiment, the Microparticle probe for single nucleotide polymorphisms may have a microrod shape.

### [Advantageous Effects]

The Microparticle probe for single nucleotide polymorphisms according to the present invention uses microparticles having higher magnetism than conventional magnetic particles (particularly magnetic beads), and thus may be mixed or moved with high efficiency using a magnet, and manufactured at a low cost. Therefore, the present invention enables the same analysis at a lower cost than existing equipment for analyzing single base polymorphisms.

In addition, the Microparticle probe for single nucleotide polymorphisms according to the present invention can accurately recognize and detect single base polymorphisms by introducing gene scissors technology.

### [Description of Drawings]

FIG. 1 is a drawing illustrating an implementation example of a microparticle probe for detecting nucleic acid according to one embodiment of the present invention.
FIGS. 2 and 3 illustrate processes for detecting a target nucleic acid using magnetic microparticles/gene scissors technology according to one embodiment of the present invention.

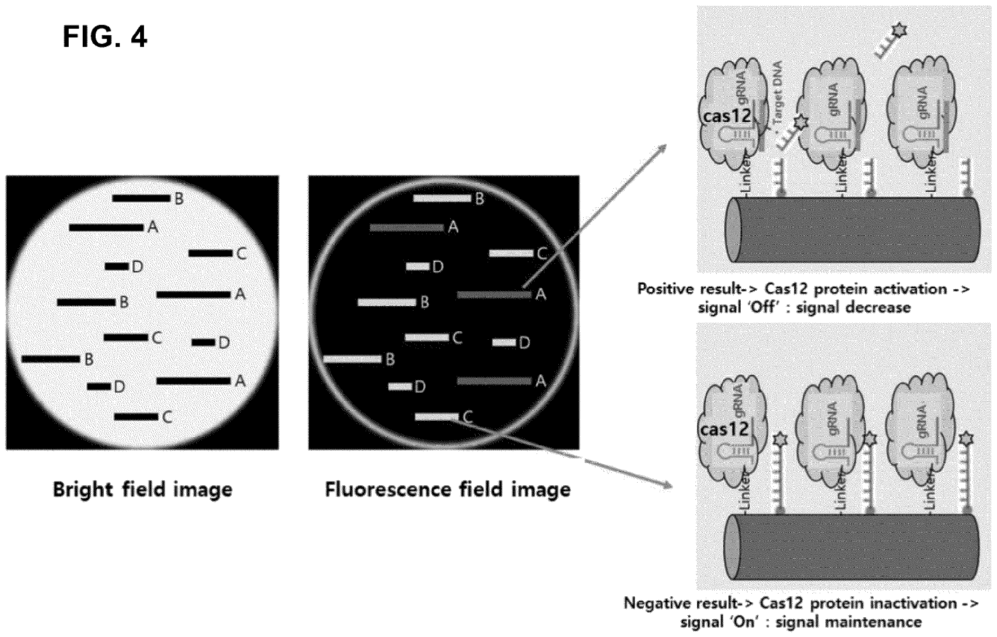

FIG. 4 virtually illustrates the results of a diagnosis according to the process by one embodiment of the present invention.
FIG. 5 illustrates the results of a preliminary verification of the possibility of capturing nucleic acids from a target sample based on magnetic particles according to one embodiment of the present invention.
FIG. 6A illustrates the capture and amplification process of target nucleic acids sequentially performed from Well 1 to Well 4 according to one embodiment of the present invention.
FIG. 6B illustrates the result of the capture and amplification process of nucleic acids for a fragment RNA and DNA (see SEQ ID NO: 1), which are arbitrary nucleic acids, according to one embodiment of the present invention.
FIG. 7 illustrates the results of confirming Cas system activity to see whether gRNA is changed in a concentration-dependent manner by magnetic particle probes coupled at different concentrations in a state where free reporter DNA and Cas protein are mixed when target nucleic acids are present.
FIG. 8 illustrates the results of an experiment according to the presence or absence of single base polymorphisms by one embodiment of the present invention.
FIG. 9 illustrates the results of measurements for the detection limit by one embodiment of the present invention.

### [Best Mode]

Hereinafter, preferred embodiments of the present invention will be described in detail. In describing the present invention, if it is determined that a detailed description of related known technologies may obscure the gist of the present invention, the detailed description thereof will be omitted. Throughout the specification, it is to be understood that the singular forms "a," "an," and "the" comprise plural referents unless the context clearly dictates otherwise, and it is to be understood that the terms such as "comprise" or "have" as used in the present specification are intended to designate the presence of stated features, numbers, steps, operations, components, parts or combinations thereof, but not to preclude the possibility of the presence or addition of one or more other features, numbers, steps, operations, components, parts, or combinations thereof. In addition, in performing the method or preparation method, each process constituting the method may occur in a different order from the specified order unless a specific order is clearly described in context. That is, each process may occur in the same order as specified, may be performed substantially simultaneously, or may be performed in the reverse order.

The technology disclosed in this specification is not limited to the embodiments described herein and may be embodied in other forms. However, the embodiments introduced herein are provided so that the content disclosed herein may be thorough and complete and the technical spirit of the present technology may be sufficiently understood by those skilled in the art. In the drawings, in order to clearly express the components of each device, the size of the components, such as width or thickness, is shown somewhat enlarged. When describing the drawings as a whole, it was described from the observer's point of view, and when one element is referred to as being located on another element, this comprises all meanings that one element may be located directly on another element or additional elements may be interposed between them. In addition, those skilled in the art will be able to implement the spirit of the present invention in various other forms within the scope that does not depart from the technical spirit of the present invention. In addition, the same reference numerals on a plurality of drawings refer to elements that are substantially the same as each other.

In this specification, the term 'and/or' comprises a combination of a plurality of recited items or any one of a plurality of recited items. In this specification, 'A or B' may comprise 'A', 'B', or 'both A and B'.

The present invention relates to a Microparticle probe for single nucleotide polymorphisms, comprising a microparticle; capture probes that are introduced to the surface of the microparticle and contain a complementary sequence to a target nucleic acid; and reporter nucleic acids that are introduced to the surface of the microparticle and generate a signal by an external stimulus.

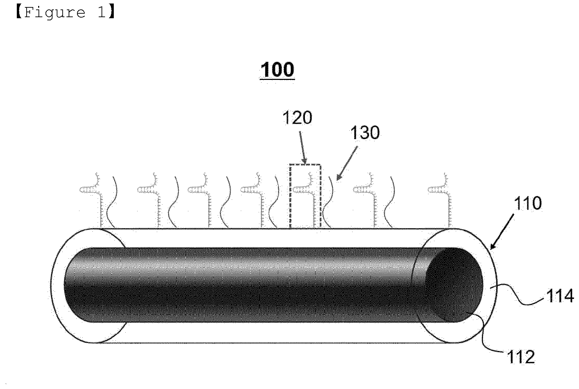

FIG. 1 is a drawing showing an implementation example of a Microparticle probe for single nucleotide polymorphisms. Referring to FIG. 1, the microparticle probe 100 for analyzing single base polymorphisms comprises a microparticle 110, capture probes 120 and reporter nucleic acids 130.

The microparticle 110 provides a surface area for detection of target nucleic acids, and for this purpose, the capture probes 120 and the reporter nucleic acids 130 can be introduced to its surface. The microparticle 110 may be made of a polymer such as polymethyl methacrylate (PMMA) or polystyrene (PS), an inorganic material such as silica, or a composite thereof. Also, to facilitate collection of detected target nucleic acids or reaction with a reagent, the microparticle 110 may contain magnetic particles inside.

The microparticle 110 may have a single structure or a core-shell structure. Preferably, the microparticle 110 may have a core-shell structure composed of a core 112 and a protective shell 114 surrounding the core. The core 112 of the microparticle 110 may comprise a magnetic substance, for example, a magneto-responsive metal, and the magneto-responsive metal may be a paramagnetic substance. Specifically, the magneto-responsive metal may be an alloy including iron (Fe), nickel (Ni), cobalt (Co), and manganese (Mn). The magneto-responsive metal may comprise a transition metal such as iron, nickel, cobalt, and manganese as a main component, and a rare earth metal such as gadolinium (Gd), terbium (Tb), and samarium (Sm), and may comprise boron (B), silicon (Si), carbon (C), and the like. Representative examples of the magneto-responsive metal comprise an iron alloy or a cobalt alloy. A specific example of the iron alloy may comprise Fe₇₀B₁₅Si₁₀C₅, and an example of the cobalt alloy may comprise Co₆₈Mn₇Si₁₀B₁₅.

Preferably, the microparticle 110 has a size and specific gravity that do not float on water so that it can be quickly collected or separated by an external magnet such as a permanent magnet or an electromagnet. In this case, the core 112 may occupy 60% or more of the total volume of the microparticle 110. For example, the volume of the core 112 may be 60 to 99%, preferably 75 to 99%, of the total volume of the microparticle 110. If the volume of the core is less than 60%, the magnetism of the core may be reduced, making it difficult to move using a magnetic bar. If the volume of the core exceeds 99%, the thickness of the shell may be reduced, resulting in reduced durability.

In addition, the microparticle 110 may have a size (e.g., diameter or length) of several tens to several hundred *µ*m, and it is preferable that the specific gravity be 5 or more. If the microparticle 110 is a nanoparticle having a size of less than 1 micrometer, it can float in water even if it is a particle having a high specific gravity (e.g., 7.876 in the case of iron). In this case, in the process of analyzing the single base polymorphisms using the microparticle 110, when a magnetic field is applied, the magnetic control of the microparticle is not smoothly performed due to the low magnetic force of the microparticle, which may cause difficulties in separation. When the magnetic rod is moved up and down within the well to promote an immune response, microparticles are attached and detached. During the process of moving the magnetic rod up and down, microparticles that are once attached to the magnetic rod may not fall back to the bottom of the well even when the magnetic field is removed due to their low weight, but may continue to remain on the magnetic rod through non-specific binding. In this case, the reproducibility of quantitative analysis may be reduced when detecting biological substances within the well.

In the case of the Microparticle probe for single nucleotide polymorphisms according to one embodiment of the present invention, its size is much larger than that of silica beads used in the conventional analysis of single base dimorphisms, and unlike conventional silica beads where magnetic particles are usually dispersed inside the silica, the magneto-responsive metal (magnetic core) occupies most of the volume of the microparticle and reacts sensitively to magnetic force, resulting in excellent reproducibility in quantitative analysis.

Meanwhile, the microparticle probe 100 for analyzing single base polymorphisms of the present invention has a magneto-responsive metal at the center and a shell layer surrounding the center, thereby forming a core-shell structure. The shell layer 114 may be made of an organic or inorganic material, and is preferably glass. In addition, capture probes such as antibodies, proteins, nucleic acids, and metabolites can be fixed on the surface of the microparticle probe 100 for analyzing single base polymorphisms. Preferably, a functional group such as an acrylic group, a hydroxyl group, an amine group, or a carboxyl group may be introduced to the surface for this purpose.

The shell layer 114 may substantially completely surround the microparticle 110, but if necessary or during the manufacturing process, the surface of the microparticle may not be completely covered by the shell layer and some areas of the surface of the microparticle may be exposed.

The thickness of the shell layer 114 may be 1 to 100 µm, preferably 1 to 50 µm, more preferably 1 to 10 µm, and even more preferably 4 to 8 µm. If the thickness of the shell layer 114 is less than the above range, the surface of the shell layer 114 may be easily broken or cracked, and if it exceeds the above range, when cutting glass, problems may arise depending on the laser characteristics and wavelength.

The core-shell structure may be formed by applying a liquid shell component to a core metal or by filling a core metal component into a hollow mold.

In this case, the shell layer 114 may be formed by solidification from a liquid coating solution of an organic or inorganic substance. More specifically, the shell layer 114 may be formed by making a liquid coating solution by melting an organic or inorganic substance-type shell component at high temperature or making it flowable or by dissolving it in a solvent, and then applying it to the core metal. The organic substance may be mainly a polymer, and the inorganic substance may be a metal or ceramic, particularly glass. For example, a coating solution obtained by dissolving plastic in a solvent or melting glass to form a shell layer 114 may be applied to the microparticle 110 in such a manner as dip coating or spray coating.

For example, when using a glass tube as the hollow mold, there are several methods, including a method of drawing a glass tube while putting metal powder into it and then melting the metal at a high temperature, a method of first drawing the glass material and then injecting a molten metal inside it, and a method of filling a glass tube with a dispersion of metal powder dispersed in an ultraviolet-curable material and then hardening it by ultraviolet irradiation. In these methods, the hollow mold itself can become a shell layer.

The microparticle 110 obtained by the above-described methods has an excellent surface uniformity. In the case of conventional particles for bioassay, a silica precursor such as TEOS is used and grown on the surface of a core particle to form a shell layer 114, but in this case, the shell layer 114 has a very rough surface. Therefore, non-specific binding of the target substance may occur a lot. This may cause unnecessary background noise.

On the other hand, the glass forming the shell layer 114, for example, borosilicate glass, can minimize non-specific reaction (non-specific binding) due to adsorption by chemical reaction with the reaction sample. In particular, the microparticle 110 according to one embodiment of the present invention has a coating layer derived from a liquid component as a shell layer 114, and therefore has a very uniform surface. The average surface roughness (Ra) of the surface of the shell layer 114 may be 15 nm or less, preferably 10 nm or less, more preferably 5 nm or less, even more preferably 2 nm or less, and especially 1.5 nm or less. In addition, Ra may be, for example, 3 nm or more, 2 nm or more, or 1 nm or more. If the surface roughness is in the above range, non-specific adsorption of the reaction sample on the surface of the shell layer 114 may be minimized.

Considering strength and transparency, the shell layer 114 of the microparticle 110 may be made of glass. The glass may be composed mainly of a compound selected from the group consisting of soda lime, borosilicate, aluminosilicate, silica, alkali silicate, Pyrex, and quartz. Preferably, for experimental purposes where heat resistance, acid resistance and water resistance are required, the glass may be borosilicate.

The microparticle 110 may have various shapes including regular shapes such as rods, flat plates, spheres, etc., or irregular shapes. Even when the microparticle 110 has a flat shape, the cross-section may have various shapes such as a star shape, a polygon shape, a circle shape, etc., and is not particularly limited. Preferably, the microparticle is in the shape of a microrod, a microdisc, or a microbead for the convenience of manufacturing and the ease of observation, and in particular, it is preferable to have a microrod shape. If the microparticle 110 has a microrod shape, it is easy to distinguish between microparticles due to overlapping, and when placed in a well, it is easy to focus, and since the area occupied by each particle is small, a large number of microparticles can be observed on a single observation screen. On the other hand, if the microparticle has a shape with a complex cross-section, such as a star shape, the edge portion may break due to collision with each other or the wall while moving inside the well, and thus it is more desirable to have a simple shape, such as a microrod.

The length of the microrod may be 10 to 1,000 µm. If the length of the microrod is less than the above range, it may not be easy to distinguish between particles of different lengths, and if the length of the microrod exceeds the above range, the particles may overlap with each other, thereby making observation difficult. In addition, the ratio of the length to the diameter of the microrod (aspect ratio) may be 2 or more, 5 or more, or 10 or more. The upper limit of the aspect ratio may be 20 or less, 10 or less, or 5 or less. If the aspect ratio is less than the above range, it may be difficult to distinguish it from a spherical microparticle as it is similar thereto, and if it is too large, it may be bent.

As a preferred embodiment, the microparticle 110 may be cut pieces of a glass-coated metal microwire. It is possible to obtain microparticles 110 having various lengths simply by cutting the glass-coated metal microwire with a laser.

The above-described microparticles can be suitably used for analyzing single base polymorphisms in a specific specimen derived from a living organism. The specimen may be a tissue extract, cell lysate, whole blood, plasma, serum, saliva, ocular humor, cerebrospinal fluid, sweat, urine, milk, ascites fluid, synovial fluid, peritoneal fluid, etc. For rapid diagnosis, the pretreatment of the sample fluid may be simplified or, in some cases, omitted.

The microparticles can be manufactured and used as particles of different sizes, lengths or shapes to analyze single base polymorphisms within a specific specimen derived from a living organism.

The capture probes 120 can be introduced into the microparticle 110 to capture biomarkers derived from a sample, particularly nucleic acid-based biomarkers. The biomarkers can be used without limitation if they are used in conventional scientific or medical fields, such as measuring or evaluating a biological process, a process causing pathogenicity, or a pharmacological process for treatment. The biomarkers may be, for example, polypeptides, peptides, nucleic acids, proteins or metabolites that can be detected in biological fluids such as blood, saliva or urine, and preferably, the biomarkers are nucleic acids in that they can be detected with high sensitivity and specificity as biomarkers associated with specific diseases.

In order to capture target nucleic acids extracted from a specimen, the microparticle according to one embodiment of the present invention may have capture probes 120 introduced onto the shell layer 114. The capture probes 120 are complementary to the target nucleic acid and specifically binds to it, thereby playing a role in fixing the target nucleic acid to the microparticle 110.

The capture probes 120 may comprise any base sequence for applying the gene scissors technology. Preferably, the capture probes 120 may be or comprise a guide RNA (gRNA). The guide RNA refers to a target DNA-specific RNA (for example, RNA capable of hybridizing with a target site of DNA) and may be used as a component of the CRISPR system together with a cleavage enzyme. The guide RNA comprises two guide RNAs, that is, a CRISPR RNA (crRNA) having a nucleotide sequence capable of hybridizing with a target site of a gene and an additional trans-activating tracrRNA (trans-activating crRNA), and may be in the form of a crRNA:tracrRNA complex (dual guide RNA) in which crRNA and tracrRNA are partially combined, or in the form of a single guide RNA (sgRNA) in which the crRNA (part or all) and the tracrRNA (part or all) are connected via a linker. The gRNA binds to the target nucleic acid, and when it meets the lyase, may cleave the target nucleic acid and the reporter nucleic acids. By utilizing these characteristics of guide RNA, it is possible to rapidly detect a biomarker by using information on changes in detection signals obtained by cutting the sequence of a specific biomarker, as will be described later. In addition, through this, it is possible to confirm that the base within the specimen has been replaced, which will be described later.

Specifically, the capture probes 120 may be a nucleic acid of 15 mer to 60 mer. In addition, it may be 20 mer to 45 mer, 25 mer to 40 mer, or 30 mer to 41 mer.

The capture probes 120 can be fixed by being absorbed or chemically linked to the surface of the microparticle 110, and may be preferably linked to a functional group on the surface of the shell layer 114. For example, biotin may be introduced to the surface of the capture probes 120 and avidin, neutravidin or streptavidin that binds to biotin may be introduced to the microparticle 110 to attach the capture probes 120 to the microparticle 110. Alternatively, the capture probe 120 may be linked to microparticle 110 using hydroxyl groups, amino groups, or carboxyl groups on the surface of microparticle 110.

If the microparticle probes for analyzing single base polymorphisms described above are used, the substitution of a single base present in nucleic acids in a sample solution can be analyzed quickly and with high sensitivity.

The reporter nucleic acids 130 are introduced to the surface of the microparticle 110 together with the capture probes 120 and generate a signal, for example, an optical or electrical signal, by external stimuli including chemical, mechanical, optical, and electrical energy. In one embodiment, the reporter nucleic acids 130 may be nucleic acids labeled with luminous substances, which may generate a luminescent signal such as fluorescence or chemiluminescence by external light or a chemical reaction.

Specifically, the reporter nucleic acids 130 may be DNA. In addition, the reporter nucleic acids may be a nucleic acid of 15 mer to 40 mer. In addition, it may be 18 mer to 30 mer, 19 mer to 25 mer, or 20 mer to 23 mer.

The luminous substances enable the detection of the presence of the target nucleic acid, by being connected to the detection probe and generating light by external stimuli such as ultraviolet rays, electron beams, chemical reactions, enzyme-substrate reactions, etc. Examples of luminous substances comprise fluorescent molecules, quantum dots, metal nanoparticles, magnetic nanoparticles, enzymes, enzyme substrates, etc. Among these, fluorescent molecules can be selected as various luminous substances in terms of ease of acquisition and application.

As examples of the fluorescent molecules, fluorescent molecules selected from the group consisting of FITC (fluorescein isothiocyanate), fluorescein, FAM (fluorescein amidite), PE (phycoerythrin), Europium, TRITC (tetramethylrhodamine isothiocyanate), Cy3 (Cyanine 3), Cy5 (Cyanine 5), Cy7 (Cyanine 7), Alexa fluorine dye, and rhodamine can be commonly used.

To detect various biomarkers within a specimen, various fluorophores such as FITC, PE, and Alexa-647 can be introduced and labeled at the 5'-position of the reporter nucleic acids 130.

In the case of microparticle probes for separation and detection of target nucleic acids according to one embodiment of the present invention, they can be applied to rapid and accurate detection of the target nucleic acid, especially substituted single base, by introducing a gene scissors system.

The reporter nucleic acids 130 can be the base sequence to be cut when the microparticle probe for detecting the nucleic acids is utilized in the gene scissors system. The gene scissors technology is a technology that causes mutations by inducing insertions, deletions, and substitutions of DNA at specific sites of the genome using a protein complex with the function of DNA nuclease. The gene scissors technology comprises Zinc Finger Nucleases (ZFNs), Transcription Activator-Like Effector Nucleases (TALENs), and the CRISPR-Cas system. Preferably, in terms of its excellent ability to accurately cut a desired portion in DNA in a simple and fast manner, the gene scissors technology is a CRISPR-Cas system. CRISPR (clustered regularly interspaced short palindromic repeats) acts as a guide RNA (gRNA) to recognize target DNA and form a complex with a lyase such as Cas endonuclease and thus to cut the DNA double strand. The CRISPR-Cas system in the present invention is not particularly limited as long as it is known, and may comprise CRISPR-Cas9, CRISPR-Cas12, CRISPR-Cas13, etc. depending on the type of lyases. Among these, in terms of having high sensitivity to mismatches in the guide RNA, the CRISPR-Cas system may preferably be CRISPR-Cas12, and more preferably, it is CRISPR-Cas12a that uses Cas12a protein as a lyase.

The CRISPR RNA may comprise a base sequence of SEQ ID NO: 1 or 2. In the case of the method for analyzing single base polymorphisms of the present invention, two types of probes are input, and then the difference in luminescence of each probe can be measured to determine whether a base is substituted. Therefore, it is desirable for the CRISPR RNA to have a specific sequence, which may be composed of two types: RNA complementary to DNA that does not show single base dimorphisms and RNA complementary to DNA that shows single base dimorphisms.

As an example, the CRISPR RNA can be the base sequence of SEQ ID NO: 1 or 2. Looking at this in detail, in the analysis of polymorphisms of CYP1A1, a mutation may occur due to a substitution of the adenine (A) site with guanine (G). In this case, unsubstituted one may be CYP1A1-A, and one that has been substituted and mutated may be CYP1A1-G. As described above, when two types of microparticle probes containing CRISPR RNA with each base sequence are injected, each probe can emit different luminescence depending on whether there is a mutation, and it is possible to perform the analysis of the single base polymorphisms by measuring this difference in luminescence.

In one embodiment, the reporter nucleic acids (130) can be attached at a high density to the surface of the microparticle 110 for reaction with Cas endonuclease (engineered nuclease) to be introduced later, which is one of the protein complexes that perform the function of DNA nuclease. The reporter nucleic acids 130 can be introduced, for example, at a density of 5 to 10×10¹⁶/m², preferably 6 to 7×10¹⁶/m², onto the surface of the microparticle 110, and in this case, a strong signal can be generated so as to easily distinguish the size and shape of the microparticle 110. The reporter nucleic acids 130 can be composed of any sequence and can have a single-stranded or double-stranded nucleic acid sequence depending on the type of Cas endonuclease. In this case, the Cas endonuclease, in an embodiment, acts by forming a complex with the assembly of the microparticle probe 100 (i.e., the state in which capture probes 120 and reporter nucleic acids 130 are uniformly distributed on the surface of the microparticle 110) and the target nucleic acid, and then cleaving reporter nucleic acids 130 distributed near the assembly of capture probes 120 and the target nucleic acid.

If the amplified product of the target nucleic acid obtained by pre-treating a specimen sample obtained from a patient is reacted with the microparticle probe 100 for separation and detection of the target nucleic acid, as described above, the complex can be formed by complementary binding with the gRNA of the capture probes of a specific target nucleic acid that recognizes the single-stranded region to be amplified. If a Cas endonuclease, such as Cas12a protein used in the CRISPR system, is applied to this complex, since the target nucleic acid is bound to the spacer sequence of the guide RNA and then the sequence cleavage function of the Cas12a protein is activated, thereby cleaving the target nucleic acid and the surrounding reporter nucleic acids 130, it is possible to observe the phenomenon in which the fluorescence signal is not maintained but is disappeared.

Therefore, by simultaneously obtaining and comparing bright field images and fluorescence field images (fluorescence images) before and after application of Cas endonuclease, the microparticles whose fluorescence has disappeared can be observed, and thus it is possible to confirm whether a specific nucleic acid is included or substituted in a specimen. By applying the gene scissors system in this way, it is possible to perform the analysis of the single base polymorphisms quickly and easily.

According to another aspect of the present invention, a kit for analyzing single base polymorphisms is provided.

The kit comprises the Microparticle probe for single nucleotide polymorphisms described above, and a lyase. The Microparticle probe for single nucleotide polymorphisms is described in detail above. As a preferred example of the lyase, Cas endonuclease can act as a component of the CRISPR-Cas system that forms a complex with guide RNA and cleaves reporter nucleic acids, and can be, for example, Cas12a protein.

In one embodiment, the kit may further comprise at least one selected from the group consisting of magnetic microparticles for analyzing single base polymorphisms, a reagent for nucleic acid amplification, a lysis solution, and a washing solution.

The magnetic microparticle for analyzing the single base polymorphisms may be silica magnetic bead or silica-coated magnetic microparticle, which is configured to have the characteristics of silica in the surface region, thereby increasing the surface area of the surface and forming a salt bridge in the presence of a high concentration of chaotropic salt contained in the lysis solution, and thus producing an effect of easily capturing nucleic acids. The reagent for amplification of the nucleic acid is not specifically limited to amplifying the target nucleic acid, but may be Loop-mediated isothermal amplification (LAMP), Single primer isothermal amplification (SPIA), Recombinase polymerase amplification (RPA), etc., in which nucleic acid amplification is performed under isothermal conditions. In terms of the characteristics of rapidity and low-temperature reaction, the RPA reagent may be preferable. For example, the RT-RPA reagent may comprise target-specific primers for specifically amplifying a gene for analyzing single base polymorphisms. The lysis solution may be a sample lysis solution based on a chaotropic salt such as a guanidinium salt. The washing solution may be an ethanol-based solution for washing the sample.

Meanwhile, the integrated equipment for driving the kit for analyzing single base polymorphisms can be combined with the kit for analyzing single base polymorphisms to configure an overall system for analyzing single base polymorphisms. That is, by providing a magnetic rod to facilitate separation and reaction of samples, an integrated equipment for operating a kit for detecting nucleic acids that can smoothly drive magnetic microparticles for analyzing single base polymorphisms in lysis samples and microparticle probes 100 for detecting signals can be comprised in the overall system.

By using the kit for analyzing single base polymorphisms, it is possible to perform the reaction through a fully automated process from the smearing of the collected sample regardless of the type of sample to the lysis of the sample, the nucleic acid capture and amplification, the capture of the amplified target nucleic acid, and the activation reaction of Cas12a due to the captured target nucleic acid, etc., and quickly and accurately detect the presence or absence of single base dimorphism by measuring the decrease in fluorescence signal due to the recognized target nucleic acids.

According to another aspect of the present invention, a method for analyzing single base polymorphisms is provided.

First, (a) a target nucleic acid is extracted from the specimen. A lysis solution containing guanidinium thiocyanate can be used to destroy the cell membrane of the specimen and release the nucleic acids inside the cells. The nucleic acids in the solution can be subjected to a process of concentrating them by attaching them to magnetic silica beads or the silica-coated microparticles 110 of the present invention and washing with alcohol, and a process of desorbing them using an elution buffer.

Next, (b) the target nucleic acid is amplified. For example, the target nucleic acid can be specifically amplified using a reverse transcription real-time polymerase chain reaction (RT-RPA) reagent.

Next, (c) the microparticle probe for analyzing base polymorphisms is provided. The microparticle probe for analyzing the single base polymorphisms comprises microparticle, guide RNA introduced to the surface of the microparticle and containing complementary sequences to the target nucleic acid, and reporter nucleic acids introduced to the surface of the microparticle and generating a signal by an external stimulus. A detailed description of each component of the microparticle is as described above.

(d) The guide RNA of the microparticle probe for separation and detection of the target nucleic acid is hybridized with the target nucleic acid to form a complex of microparticle probe-nucleic acid for separation and detection of the target nucleic acid.

In one embodiment, the microparticle probes for analyzing single base polymorphisms can be immobilized with guide RNA targeting only specific viruses and reporter DNA attached with a 5'-fluorophore. The gRNA of specific target magnetic particle probe that recognizes the single-stranded region to be amplified can form a complex by complementary binding with a gene showing single base polymorphisms. The formation of the complex may comprise a process of promoting the reaction using an external magnetic force. For example, to promote the reaction, the well containing the reactants can be heated to an appropriate temperature, and the reaction can be promoted by continuously moving the magnetic rod up and down inside the well to control microparticles containing the magnetic substance.

(e) The lyase is coupled to the guide RNA of the microparticle probe for separation and detection of the target nucleic acid. A preferred example of the lyase is Cas endonuclease, for example, Cas12a.

The lyase recognizes and binds to the scaffold sequences of gRNA immobilized on the microparticle probe for separation and detection of the target nucleic acid.

(f) Afterwards, each base sequence of the target nucleic acid and the reporter nucleic acids is cleaved by the activation reaction of the lyase. If the amplified target nucleic acid is bound to gRNA, the Cas protein is activated and each base sequence of the target nucleic acid and the reporter nucleic acids can be cleaved.

Meanwhile, the microparticle probe for which the reaction is completed can be washed with a washing buffer, and preferably, a washing process of the microparticle probe using an external magnetic force can be comprised. That is, after the reaction is completed, a step of continuously washing the microparticle probe for analyzing base polymorphisms in two or more washing wells using a magnetic bar can be further comprised. The product is washed. After washing, the luminescence signal from the microparticle probe can be detected to accurately confirm whether the single base polymorphism is present.

(g) The target nucleic acid is detected by measuring the on-off of the signal emitted from the complex by the external stimulus. The signal may be a luminescence signal emitted from a luminescent substance in the complex. The external stimulus can be ultraviolet rays, electron rays, chemical reactions, enzyme-substrate reactions, etc. For example, before treatment with Cas endonuclease, all microparticle probes can be turned on by fluorescence generated by fluorescent molecules bound to the reporter nucleic acids by external stimulus such as ultraviolet light. Afterwards, when a Cas endonuclease is bound to the guide RNA, the base sequences of the reporter nucleic acids together with the target nucleic acid are cleaved by the Cas endonuclease, thereby turning off the fluorescence of the microparticle probe to which the target nucleic acid is attached. By overlaying the fluorescence image with the bright field image and analyzing it, the microparticle probe that is complexed with the target nucleic acid can be identified, thereby allowing the target nucleic acid to be distinguished.

In that case, the detection of the target nucleic acid can be performed by measuring the difference in luminescence of each probe using two or more types of microparticle probes for analyzing single base polymorphisms, thereby identifying the presence or absence of the target nucleic acid. That is, in the case where there are nucleic acids where single base substitution has not occurred and the case where there are nucleic acids where single base substitution has occurred, since different types of probes emit light, respectively, these can be distinguished by taking bright field images and fluorescence field images in parallel.

In addition, by configuring the two or more types of probes in a state where capture probes that specifically bind to each nucleic acid are fixed as discussed above, analysis of single base polymorphisms can be easily performed. For example, if the microparticle probe is a microrod, the type and amount of each nucleic acid can be quickly and independently obtained through the process of analyzing bright field images and fluorescence field images for base-substituted and unsubstituted nucleic acids by using microrods with different lengths and utilizing gene scissors technology such as the CRISPR-Cas system. For example, if microrods made by cutting glass-coated microwires into various lengths are used as microparticle probes, analysis for single base polymorphisms is possible with just one fluorescent substance. By interpreting microparticle probes encoded with each length information through image analysis, multi-detection is possible at once, and there is an advantage that it can maintain the accuracy of high sensitivity.

That is, if two or more types of microparticle probes for analyzing single base polymorphisms are used, it is possible to analyze single base polymorphisms for various target nucleic acids through a single detection process.

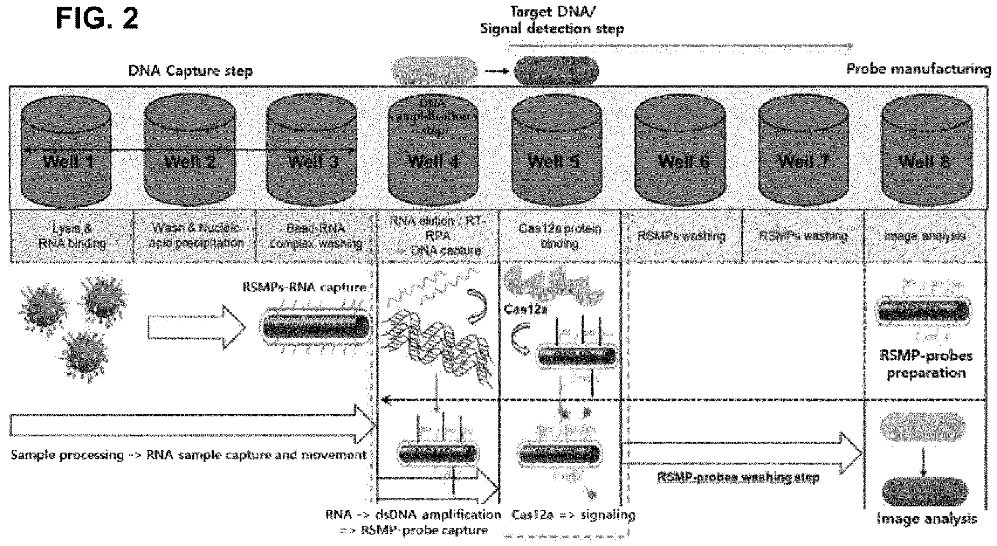

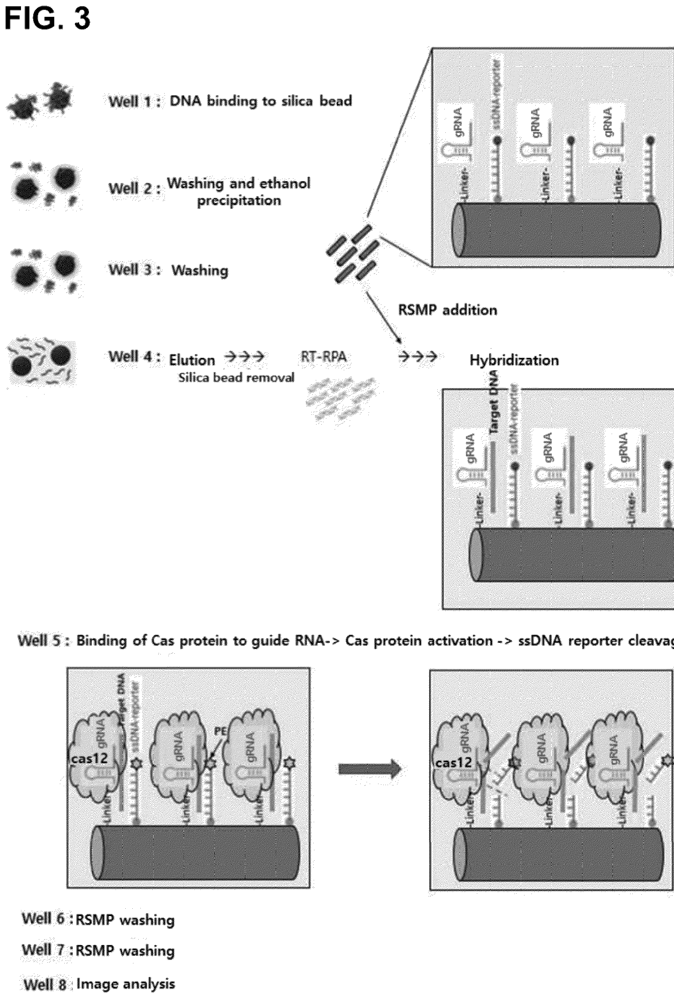

FIGS. 2 and 3 illustrate processes for detecting the target nucleic acid using magnetic microparticle/gene scissors technology. Referring to FIGS. 2 and 3, the processes for detecting target nucleic acids are shown, which are performed in eight wells (Well 1 to Well 8), wherein a DNA capture step is performed in Well 1 to Well 3, a DNA amplification and hybridization step is performed in Well 4, and an image analysis step is performed in Well 5 to Well 8. The procedure in each well is described in more detail as follows.

### 1) Well 1: Binding of DNA to silica beads

The specimen is placed in well 1, which contains magnetic silica bead, chaotropic salt, and lysis solution, and thus the cell membrane of the specimen is destroyed and the nucleic acid within the cells is released, and then the nucleic acid is bound to the magnetic silica bead by the action of the chaotropic salt.

### 2) Well 2: Washing and precipitation in ethanol

The magnetic silica bead to which nucleic acids are bound is transferred to well 2, and the reaction of DNA and salts is enhanced by 33% isopropanol, so that the DNA is strongly attached to the magnetic silica bead, and unnecessary residual substances are removed.

### 3) Well 3: Washing

The magnetic silica bead to which nucleic acid is bound is washed using 70% ethanol. During this process, any residues other than nucleic acids are additionally removed as much as possible.

### 4) Well 4: Nucleic acid amplification and hybridization

The magnetic silica bead to which the nucleic acid is bound is transferred to well 4 containing the elution buffer to detach the nucleic acid from the bead. Next, cDNA is synthesized and amplified using the RT-RPA reagent contained in well 4. The RT-RPA reagent may contain target-specific primers for specifically amplifying the target nucleic acid for analyzing single base polymorphisms.

Meanwhile, a guide RNA that targets only a specific base sequence and a reporter DNA in the form of single-stranded random DNA with a 5'-fluorophore attached for signal detection are fixed to each magnetic microparticle probe of a specific length. As the base of the nucleic acid to be analyzed is amplified by RT-RPA, the gRNA of specific target magnetic particle probe that recognizes the single-stranded region that is amplified in this process binds complementarily to the nucleic acid to be analyzed, thereby forming a complex of the magnetic microparticle probe and the genetic sequence of the nucleic acid to be analyzed.

### 5) Well 5: Binding of Cas protein and cleavage of reporter DNA sequence

A magnetic microparticle probe, which is round-shaped magnetic particle (RSMs) whose reaction is completed in well 4, is transferred to well 5, which contains Cas12a endonuclease. The Cas12a protein recognizes and binds to the scaffold sequence of the gRNA that is immobilized on the magnetic particle probe. If the gRNA-cas12a protein complex is formed and the target DNA is amplified in well 4 and bound to the spacer sequence of gRNA, the endonuclease function of Cas12a protein is activated to cleave the target DNA and surrounding non-specific DNA. Conversely, if the target DNA is not amplified in well 4, the gRNA-cas12a protein complex is formed in well 5, but the target DNA is not bound to the spacer sequence of guide RNA, and thus the endonuclease function of Cas12a protein is not activated.

### 6) Well 6: Washing

The magnetic microparticle probe, after the reaction is complete, is washed with washing buffer.

### 7) Well 7: Washing

The magnetic microparticle probe, after the reaction is complete, is washed once more with washing buffer.

### 8) Well 8: Image analysis

The magnetic microparticle probe, after the reaction and washing are completed, is imaged. At this time, both bright field image and fluorescence field image are acquired.

### 111

The Microparticle probe for single nucleotide polymorphisms described above and the method for analyzing single base polymorphisms using the same can have the following advantages. The present invention overcomes the limitations of the existing method for analyzing single base polymorphisms using the existing Reverse Transcriptase-Polymerase Chain Reaction (RT-PCR) method by using the CRISPR-CAS gene scissors technology. The existing molecular diagnostic method for analyzing single base polymorphisms involves the process of collecting specimens on-site, extracting genes from the accession institution, amplifying them, and detecting them, and uses an expensive equipment to obtain the results of gene amplification. The existing method is not suitable for testing a large number of samples on-site due to this process, and it takes at least 6 hours or more to obtain the results. However, the present invention enables the process of gene extraction, amplification, and detection on-site and may be automated by introducing the CRISPR-CAS gene scissors technology (the time in the gene extraction and amplification steps may be significantly reduced by using magnetic particles). In addition, the present invention overcomes the non-specific amplification phenomenon seen in existing molecular diagnosis by using CRISPR-Cas gene scissors technology.

The present invention, which introduces microparticle probes/gene scissors technology for analyzing single base polymorphisms, recognizes and operates the exact sequence of a gene, thereby increasing sensitivity and specificity compared to existing molecular diagnostic technologies. Additionally, the time required for analyzing single base polymorphisms may be reduced to 30 to 40 minutes.

Hereinafter, preferred embodiments of the present invention will be described with reference to the accompanying drawings so that those skilled in the art can easily practice them. Also, in describing the present invention, if it is determined that a detailed description of related known function or known configuration may obscure the gist of the present invention, the detailed description thereof will be omitted. In addition, certain features presented in the drawings have been enlarged, reduced or simplified for ease of explanation, and the drawings and their components are not necessarily drawn to proper scale. However, those skilled in the art will readily understand these details.

### Examples

In this example, the experimental steps that are performed as a single process are arbitrarily divided into a step of lysing the target factor and capturing and amplifying nucleic acids, a step for producing magnetic particle probes for signal detection, and a step for detecting signals according to the reaction of the target factor, etc., and each step is described in detail.

### Example 1. Preparation of magnetic microparticles for capturing nucleic acid

In this system for analyzing single base polymorphisms, eight wells were used, and in order to move nucleic acids between each well, it is essential to use magnetic microparticles that can stably capture nucleic acids extracted from target factors and move them between wells. For stable capture of nucleic acids, cylindrical magnetic microparticles or commercially available magnetic microparticles with a size of 10 to 1000 *µ*m were used as raw materials to manufacture magnetic particles with sufficient surface area for capturing nucleic acids.

First, the prepared basic magnetic microparticles (Manufactured by our company; 100-500 *µ*m-long cylindrical rod-shaped microparticles as basic magnetic microparticles in a core-shell form without capture probes corresponding to 110 in FIG. 1) were washed three times with a 0.1 M sodium hydroxide solution and three times with a 100% ethanol solution, and then washed by ultrasonic waves for 2 seconds.

Next, 200 mg of the magnetic microparticles were added to 10 ml of a solution containing ammonia water (composition of 20 ml of 100% ethanol, 3 ml of deionized water, and 1 ml of ammonia water), and 2 ml of tetraethylorthosilicate (TEOS) solution was added based on 100 *µ*ℓ of TEOS per 10 mg of the magnetic particles. The magnetic microparticles were produced by performing the reaction four times in total while adding 2 ml of TEOS solution for each reaction, provided that a process of rotating the solution at 20 rpm for 20 minutes using a rotator device (FINEPCR, Hybridization Incubator Combi-H12) was set as one reaction. The magnetic particle obtained after the reaction was washed three times with the previously mentioned ammonia water solution and three times with 100% ethanol, and then stored in an ethanol solution.

In order to verify the ability of magnetic microparticles manufactured to capture nucleic acids, random samples were prepared to verify the actual ability to capture nucleic acids. Although a virtual virus or harmful bacteria can be assumed as a random sample, in this example, a random E. coli was selected as a target and the presence or absence of nucleic acid capture was confirmed.

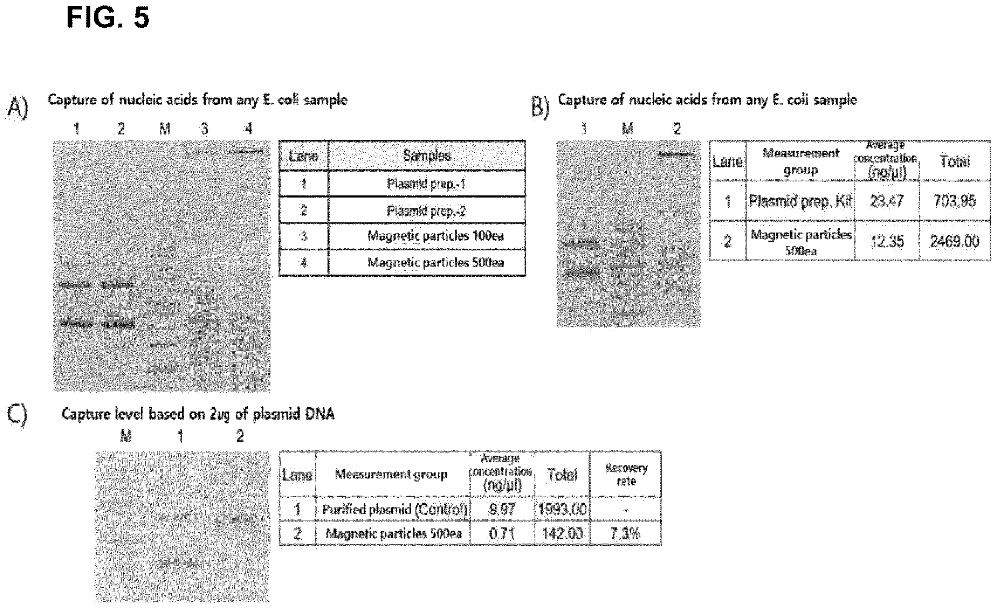

FIG. 5 illustrates the results of preliminary confirmation of the possibility of nucleic acid capture from a target sample based on magnetic particles. A) in FIG. 5 is a comparison of the results of purifying and separating plasmid DNA from E. coli, into which plasmid DNA was introduced, on the basis of the existing commercial E. coli plasmid mini prep. kit (Bioneer, AccuPrep^{®} Plasmid Mini Extraction Kit) based on E. coli samples set as arbitrary target factors, and the results of purifying and separating plasmid DNA from E. coli, into which plasmid DNA was introduced, by using agarose gel-based electrophoresis through the magnetic microparticle beads for nucleic acid capture manufactured based on our magnetic microparticles.

Referring to A) in FIG. 5, it can be confirmed that our magnetic particles for purifying nucleic acids can purify and capture plasmid DNA under conditions of 100 and 500 particles.

Lysis buffer: Based on the buffer composition (2M guanidinium thiocyanate, 36.7 mM Tris-Cl, 16.7 mM EDTA, 2% Triton X-100, 33% isopropanol, RNase free water) of Well 1, after mixing 1 ml of cultured E. coli concentrated sample and our magnetic microparticle beads, 10 minutes of rotational stirring was performed, lane 1, 2 - purification results 1, 2 by commercial plasmid prep. kit, lane 3 - purification results based on 100ea of our nucleic acid purification beads, lane 4 - Purification results based on 500ea of our nucleic acid purification beads, lane M: 1kb DNA ladder (Solgent, 1Kb Plus DNA Ladder)).

B) in FIG. 5 shows the results of nucleic acid purification confirmed based on 500 ea of nucleic acid purification beads of our company and the plasmid prep kit under the same conditions as A) in FIG. 5. Referring to B in FIG. 5, it can be confirmed that compared to the plasmid prep. kit, both genomic DNA of E. coli and plasmid DNA can be purified and recovered through our magnetic particle nucleic acid purification.

C) in FIG. 5 shows the results confirming the capture ability of our magnetic particles targeting plasmid DNA at a concentration of 2 *µ*g. Referring to C) in FIG. 5, the ability to capture any nucleic acid can be confirmed by the fact that our magnetic particles for nucleic acid purification can capture nucleic acid samples at a rate of approximately 7.3%.

### Example 2. Steps of lysis of target factor and capture and amplification of nucleic acid (Well 1 - Well 4)

The applicability of our automated equipment, which performs a single process from lysis of target samples to imaging of magnetic particle probes, was confirmed. A mobility test was conducted to confirm whether the target nucleic acids obtained by lysis and capture undergo a sequential process through normal movement between wells.

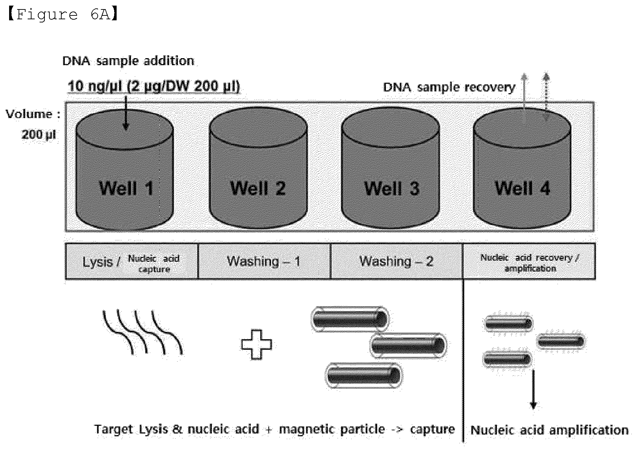

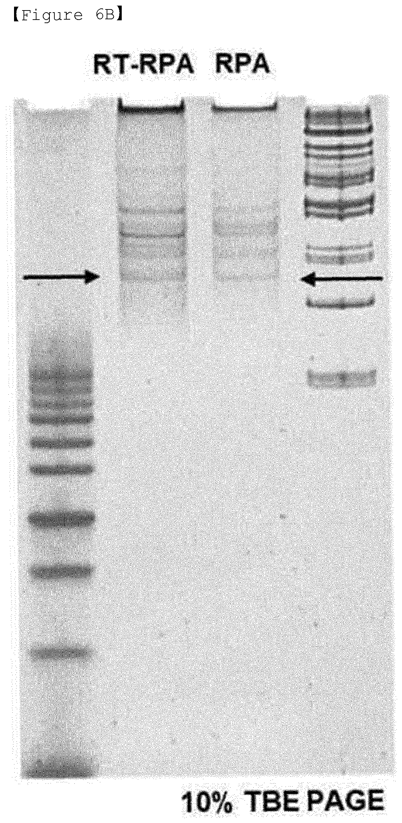

FIG. 6 illustrates the results of confirming the capture of nucleic acids from a target sample and the amplification of target site nucleic acids in a multiplex diagnostic method according to one embodiment of the present invention.

FIG. 6A illustrates the capture and amplification process of target nucleic acids sequentially performed from Well 1 to Well 4. Referring to FIG. 6A, lysis/nucleic acid capture was performed in well 1, and nucleic acids were extracted and captured from the virus sample using a solution (2M guanidinium thiocyanate, 36.7 mM Tris-Cl, 16.7 mM EDTA, 2% Triton X-100, 33% isopropanol, RNase free water) containing chaotropic salts for extraction and capture from the virus sample.

The first washing process (washing-1) was performed in well 2, where the magnetic particles containing captured nucleic acids were washed using 2M guanidinium thiocyanate, 36.7 mM Tris-Cl, 16.7 mM EDTA, 2% Triton X-100, RNase free water + 66% Isopropanol, and the captured nucleic acids were further concentrated in the magnetic particles.

The second washing process (washing-2) was performed in Well 3, and 70% ethanol was used to remove the remaining waste attached to the magnetic particles.

Recovery and amplification of nucleic acids were performed in Well 4. After detaching the nucleic acids attached to the magnetic particles, the detached nucleic acids were amplified. A solution based on the Recombinase Polymerase Amplification (RPA) enzyme complex was used for the amplification of nucleic acids.

FIG. 6B illustrates the results of the nucleic acid capture and amplification process for arbitrary nucleic acids, such as fragment RNA and DNA (see SEQ ID NO: 1). The target nucleic acids detached in Well 4 were subjected to Reverse Transcriptase Recombinase Polymerase Amplification (RT-RPA) and RPA reactions at 37°C for 20 minutes, under the condition where any fragment of nucleic acid RNA or DNA was introduced. The composition of the RPA reagent used at this time was carried out according to the protocol recommended by the manufacturer (TwistDx, TwistAmp Liquid Kit). Referring to FIG. 6B, it was confirmed that after each RNA sample and DNA sample was captured and moved between the wells, the nucleic acids were dissociated in well 4 and then amplified normally by RT-RPA and RPA enzyme complexes. TBE PAGE gel electrophoresis was performed for the purpose of more clearly confirming the size of amplified nucleic acids less than 700 bases.

Table 1 provides information about targets for testing single base polymorphisms.

**Table 1:**

| |
|---|
| + 293 cell (human embryonic kidney cell) gDNA (CYP1A1-A) |
| + Mutant (plasmid, CYP1A1-G) |

Table 2 below illustrates primers (115 bp) for amplification of the target sequence.

**Table 2:**

| CYP1A1 | |
|---|---|
| Forward | 5' -AAGAGAAAGACCTCCCTTTGGGCAA-3' (SEQ ID NO: 1) |
| Reverse | 5'-CACCCCTGATGGTGCTATCGACAAG-3' (SEQ ID NO: 2) |

Table 3 below illustrates the information on Guide RNAs.

**Table 3:**

| uA-crRNA |
|---|
| 5'-UAAUUUCUACUAAGUGUAGAUCAUUGUCUCACCGAUACAC-3' (SEQ ID NO: 3) |

| uG-crRNA |
|---|
| 5' -UAAUUUCUACUAAGUGUAGAUCACUGUCUCACCGAUACAC-3' (SEQ ID NO: 4) |

Table 4 below illustrates Reporter DNAs for fluorescent signal detection.

**Table 4:**

| | | |
|---|---|---|
| 1 | For Reporter DNA QC (Attachment of quencher) | 5'-TAMRA-TTA TTA TT-3'-BHQ2 (SEQ ID NO: 5) |
| 2 | For attachment of Reporter DNA-Fluorphore | 5'-Thiol-TTA TTA TT-3'-NH2 (SEQ ID NO: 5) |
| 3 | Reporter DNA-TAMRA | 5' -NH2-TTA TTA TTT T-3'-TAMRA (SEQ ID NO: 6) |

Based on the above results, it was indirectly confirmed that it is possible to detect target signals and analyze single base polymorphisms based on amplified nucleic acids.

### Example 3. Fabrication of a magnetic particle probe for signal detection and verification of its functionality (Well 4 ~ Well 5)

In the magnetic particle probes for signal detection, guide RNAs (gRNAs), which recognize the target site sequence of the target factor, and the gRNAs/target sequence/Cas12a complex play a role in cleaving the sequence. In that case, the magnetic particle probes are produced in a state where single-stranded reporter DNAs, which play a role in detecting signals, are mixed. Therefore, when gRNAs, which play a role in recognizing target site sequences, are mixed with reporter DNAs and coupled to the magnetic particles, it is necessary to confirm whether they can play the role of gRNAs.

An experiment was designed to confirm the coupling results of gRNAs or fluorescently labeled reporter DNAs to magnetic particles alone. The sequence information of gRNAs and reporter DNAs can be found in SEQ ID NO: 3 to SEQ ID NO: 6 in the sequence listing.

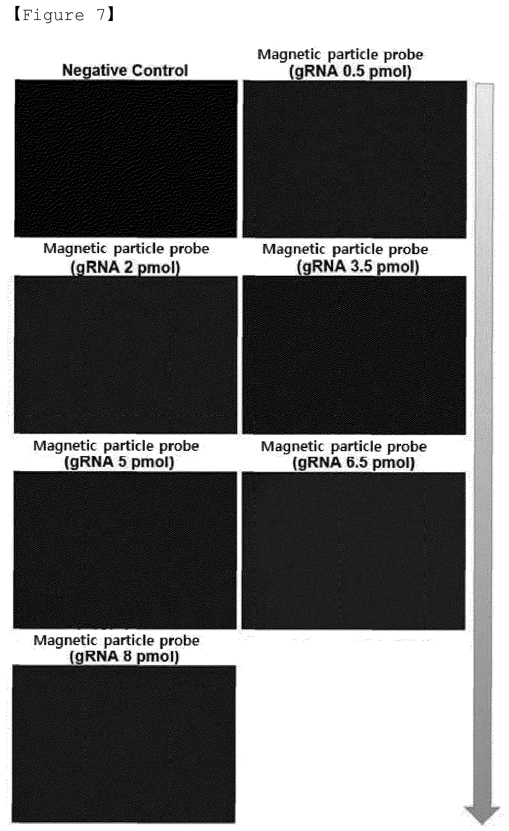

FIG. 7 illustrates the results of confirming Cas system activity to see whether gRNA is changed in a concentration-dependent manner by magnetic particle probes coupled at different concentrations, in a state where free reporter DNA, Cas protein, is mixed in the presence of target nucleic acids. This experiment was conducted for the purpose of verifying the concept of the Cas system. For constitution of free reporter DNAs, a fluorescent factor was bound to one side, and a quencher that absorbs the fluorescent signal was bound to the other side, and then the test was conducted. Referring to FIG. 7, if the gRNA sequence is coupled to the surface of the magnetic particle depending on the concentration, it was confirmed that the free reporter DNA, in which the functionality of the fluorescent factor was masked by the quencher, is cleaved at the quencher site depending on the concentration of gRNA, and a fluorescent signal is detected.

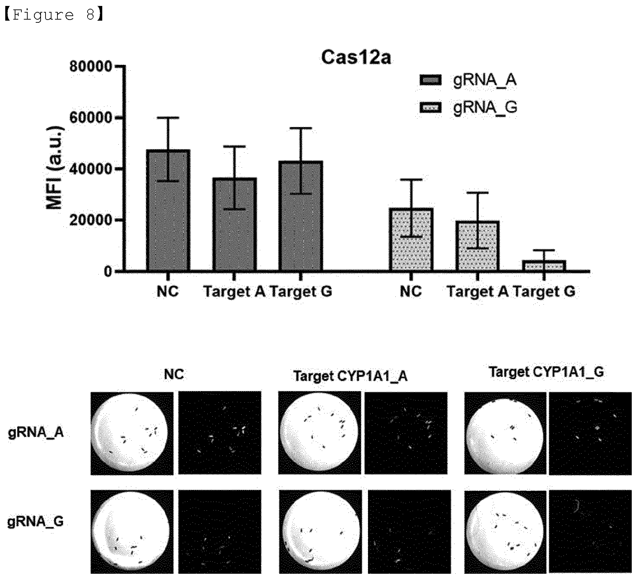

FIG. 8 illustrates the results of an experiment according to the presence or absence of single base polymorphisms. If bases that do not show single base polymorphisms are used (gRNA_A), it was confirmed that the observation of probes designated as Target A is decreased, and it was also shown that this can be distinguished when taking a bright field image and a fluorescence image. However, since these polymorphisms do not appear in most base, it was judged that the change in light quantity is very small as compared to NC, which can minimize false positives.

In addition, the bases that show single base polymorphisms are used (gRNA_G), it was confirmed that the overall amount of fluorescence was very low because it was observed only in some bases. In particular, it was found that the observation of probes designated as Target G is very weak, making it easy to distinguish them.

Through these, it can be seen that the amount of light from the probes is greatly changed only when the polymorphisms appear, and thus it was confirmed that the single base polymorphisms can be accurately analyzed while minimizing false positives.

### Example 4. Verification of the detection limit of a magnetic particle probe for signal detection

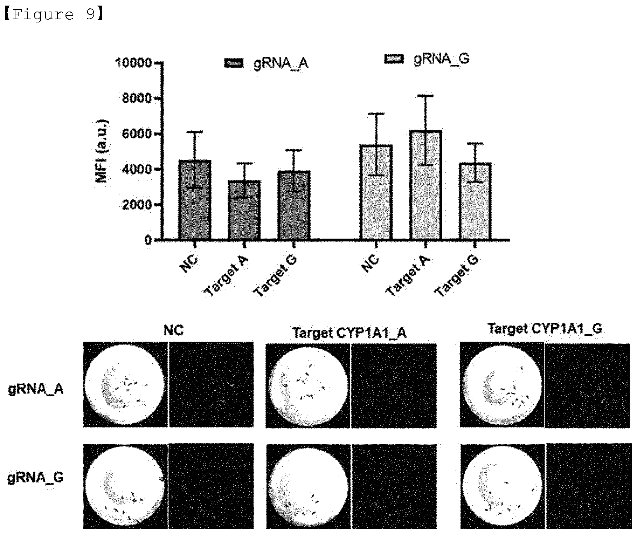

In the case of FIG. 9, the detection limit was confirmed by reducing the amount of gRNAs used by 1/10 compared to the above Example 3. In the case of Example 3, the amount of gRNAs injected was 0.0125 pmol/ul, but in the case of Example 4, 0.00125 pmol/ul was used, thereby measuring the detection limit. As a result, as shown in Table 9, it was found that the change in the amount of light is greater when there are bases showing polymorphisms similar to Example 3.

That is, in the case of the present invention, it was found that it has a detection limit of 0.00125 pmol/ul, which means that even when using a minimal sample, it is easy to analyze whether there are single base polymorphisms.

As described above, although specific parts of the present invention have been described in detail, it will be apparent to those skilled in the art that such specific descriptions are merely preferred embodiments and are not intended to limit the scope of the present invention. Accordingly, the substantial scope of the present invention will be defined by the appended claims and their equivalents.

## Claims

1. A Microparticle probe for single nucleotide polymorphisms, comprising,
a microparticle;
capture probes that are introduced to the surface of the microparticle and contain a complementary sequence to a target nucleic acid; and
reporter nucleic acids that are introduced to the surface of the microparticle and generate a signal by an external stimulus.

2. The microparticle probe according to claim 1, wherein the capture probes contain a guide RNA.

3. The microparticle probe according to claim 2, wherein the guide RNA comprises CRISPR RNA (crRNA) having a nucleotide sequence capable of hybridizing with a target site of a gene and trans-activating crRNA (tracrRNA).

4. The microparticle probe according to claim 2, wherein the guide RNA binds to the target nucleic acid, and when it meets the lyase, cleaves the target nucleic acid and the reporter nucleic acids.

5. The microparticle probe according to claim 1, wherein the reporter nucleic acids are nucleic acids labeled with a luminous substance.

6. The microparticle probe according to claim 1, wherein the reporter nucleic acids become the base sequences to be cut when used in the gene scissors system.

7. The microparticle probe according to claim 1, wherein the microparticle contains magnetic particles inside.

8. The microparticle probe according to claim 1, wherein the microparticle has a core-shell structure having a core containing a magnetic substance and a shell layer having a uniform thickness surrounding the core.

9. The microparticle probe according to claim 1, wherein the microparticle has a size and specific gravity that do not float in water.

10. A kit for analyzing single base polymorphisms, comprising the Microparticle probe for single nucleotide polymorphisms according to any one of claims 1 to 9, a lyase, and a reagent for amplifying nucleic acids.

11. A method for analyzing single base polymorphisms, comprising the steps of,
(a) extracting a target nucleic acid for analyzing single base polymorphisms from a specimen;
(b) amplifying the target nucleic acid;
(c) providing a Microparticle probe for single nucleotide polymorphisms comprising a microparticle, a guide RNA that is introduced to the surface of the microparticles and contains complementary sequences to the target nucleic acid, and reporter nucleic acids that are introduced to the surface of the microparticles and generate a signal by an external stimulus;
(d) reacting the guide RNA of the microparticle for analyzing the single base polymorphisms with the target nucleic acid to form a complex of the Microparticle probe for single nucleotide polymorphisms and the target nucleic acid;
(e) binding lyase to the guide RNA of microparticles for analyzing the single base polymorphisms;
(f) cleaving each base sequence of the target nucleic acid and the reporter nucleic acids by an activation reaction of the lyase; and
(g) detecting the target nucleic acid by measuring the on-off of the signal emitted from the complex by the external stimulus.

12. The method for analyzing single base polymorphisms according to claim 11, wherein the detection of the target nucleic acid is to use the microparticle probe for analyzing two or more types of single base polymorphisms and measure the difference in luminescence of each probe to identify the presence or absence of the target nucleic acid.

13. The method for analyzing single base polymorphisms according to claim 11, wherein the microparticle probe for analyzing the single base polymorphisms has the form of microrods.
